# EUROPÄISCHE PATENTANMELDUNG

(11) **EP 0 834 294 A1**
(43) Veröffentlichungstag der Anmeldung: **08.04.1998**
(21) Anmeldenummer: 96810672.4
(22) Anmeldetag: 07.10.1996
(51) Int. Cl.: A61F 2/38, A61F 2/30

(54) **Baukastensystem für zementierte Prothesen**

(71) Anmelder: Sulzer Orthopädie AG, 6340 Baar (CH)
(72) Erfinder: Sandoz, Yvan, 8405 Winterthur (CH); Seidl, Alex, 8055 Zürich (CH)
(74) Vertreter: Heubeck, Bernhard

(57) **Zusammenfassung**

Mit der Erfindung werden flächige Distanzstücke für in Knochen zementierbare Prothesenteile gezeigt, die es ermöglichen ein Prothesenteil mit Lagekorrektur seiner Funktionsflächen einzuzementieren, ohne zusätzliche Risiken beim Zementieren zu erzeugen. Dadurch, dass die Distanzstücke selbst aus ausgehärtetem Knochenzement bestehen, entsteht mit dem flüssigen Knochenzement eine feste homogene Bindung. Gleichzeitig erlauben diese Distanzstücke auch beim Zementieren eine feine, baukastenartige Abstufung und ein exaktes Positionieren von Prothesenteilen.

## Beschreibung

Die Erfindung handelt von einem Baukastensystem für zementierte Prothesen, insbesondere für zementierte Knieprothesen, bei denen ein Prothesengelenkteil mit Knochenzement an einem resektierten Knochen befestigbar ist.

Bei zementierten Prothesenteilen wird das Teil mit Knochenzement an einem resektierten Knochen befestigt, wobei der Knochenzement an Knochen und Prothese in Vor- und Rücksprünge einfliesst und mit dem Aushärten beide Teile gegeneinander verankert. Um die Funktionsflächen von Prothesenteilen trotz unterschiedlicher Resektion des Knochens am richtigen Ort zu plazieren, sind baukastenmässig verschiedene Füllstücke entwickelt worden, die den Abstand zwischen resektierter Fläche und der Funktionsfläche in Stufen vergrössern können.

Bei einer metallischen Tibiaplattform die mit Knochenzement an einer Tibia befestigbar ist, sind unterschiedlich dicke Zwischenplatten zwischen Plattform und Lagerschale möglich. Die Befestigung der Lagerschale über ein solchen Zwischenstück ist jedoch so heikel, dass viele Hersteller es vorziehen unterschiedlich hohe Lagerschalen einzusetzen.

Eine weitere Möglichkeit besteht in unterschiedlich dicken Zwischenplatten, die auf der Unterseite der Tibiaplattform mit Schrauben befestigt werden, und die mit der an ihnen befestigten Plattform einzementierbar sind. So hat die Firma Smith & Nephew Richards Inc., 1450 Brooks Rd., Memphis, TN 38116, USA, metallische Zwischenplatten unterschiedlicher Dicke mit strukturierter Oberfläche entwickelt, die sowohl zur Plattform als auch zum Knochen hin mit Knochenzement verankerbar sind. Solche Verankerungen haben den Nachteil, dass zusätzlich zur Verankerung zwischen Knochenzement und Tibiaplattform zwei weitere Verankerungen mit schwalbenschwanzähnlichen Vor- und Rücksprüngen von der Zwischenplatte zum Knochenzement notwendig sind. Dabei hängt es vom Geschick des Operateurs ab, dass der Knochenzement wirklich in die Hinterschneidungen an der Zwischenplatte einfliesst.

Die Erfindung hat die Aufgabe, einen Baukasten mit sicherer Verbindung zwischen Zwischenplatte und Knochenzement herzustellen. Diese Aufgabe wird mit den Kennzeichen vom unabhängigen Anspruch 1 gelöst, indem das Baukastensystem mindestens ein vorgefertigtes flächiges Distanzstück aus ausgehärtetem Knochenzement aufweist, das auf seinen flächigen Seiten mit Knochenzement am Knochen respektive am Prothesenteil befestigbar ist, um den Abstand zwischen Prothesenteil und Knochen zu vergrössern.

Versuche haben gezeigt, dass derartige Distanzstücke, die in unterschiedlichen Dicken und Grössen auf Vorrat gehalten werden können, sich vollständig an ihrer Oberfläche mit dem noch flüssigen Knochenzement verbinden. So wird beispielsweise bei Distanzstücken aus PMMA die Oberfläche durch einen kurzzeitig im flüssigen Zustand noch benetzenden Knochenzement aus PMMA soweit angelöst, dass auch bei völlig glatten Flächen ein homogener Uebergang im Material entsteht. Ein solches Distanzstück hat den Vorteil, dass bezüglich der Sicherheit für die Befestigung der zementierten Prothese nicht mehr Risiko besteht, als wenn die Prothese direkt einzementiert wird. Im Gegenteil, es genügen kleine Zementmengen für die Befestigung, die Lage der Prothese ist sehr genau vorbestimmbar und die Prothese kann sich auch bei flüssigem Zement kaum verschieben. Dieser Effekt wird durch eine Strukturierung der Oberfläche vom Distanzstück noch verstärkt, indem Vorsprünge, die auf der Gegenseite aufliegen und dort eventuell noch zentriert werden, dafür sorgen, dass eine gleichmässige und dünne Zementschicht entsteht. Dabei können die Vorsprünge als relativ dünne Stege oder Noppen ausgeführt sein, die als Wegbegrenzung beim Anpressen der Prothese dienen.

Im weiteren können mit umlaufenden Kanten flache Hohlräume gebildet werden, in die der flüssige Zement einfüllbar ist, um ihn unabhängig von der Richtung der Schwerkraft zur Gegenfläche einzuschliessen und um überflüssigen Zement abzuquetschen. Die Lage der Prothese lässt sich so vor dem eigentlichen Zementieren durch Messen oder durch den Distanzstücken nachgebildete Probierstücke sehr genau variieren und überprüfen. Dadurch, dass die Variation des Abstandes der Funktionsflächen der Prothese zum resektierten Knochen in ein "Billigteil" verlegt wurde, das keine zusätzlichen Risiken in sich birgt, ist ein Vorrat von fein abgestuften Dicken von Distanzstücken möglich. Da bei der Resektion eines Knochens auch die Knochenbeschaffenheit und eine nicht zu vermeidende Streuung eine Rolle spielen, können neben Distanzstücken mit parallelen flächigen Seiten auch Distanzstücke mit keilförmig zueinander stehenden flächigen Seiten zum Ausgleich von Vorteil sein. Ein weiterer Vorteil ergibt sich, wenn die Distanzstücke über Hilfsflächen zum Prothesenteil zentrierbar sind und so über die Prothese bis zum Aushärten des Knochenzements am vorgesehenen Ort anpressbar sind. Eine Zentrierung kann auch entstehen, wenn Distanzstück und Gegenfläche in mehreren Ebenen abgewinkelt sind oder wenn die flächigen Seiten Ausschnitte von Kugelflächen sind.

Die Herstellung der Distanzstücke aus flüssigem Knochenzement kann durch Giessen, Spritzen oder durch Warmpressen von Halbfabrikaten erfolgen, wobei das Herstellverfahren den Stückzahlen angepasst ist. Da eine spanende Formgebung nicht notwendig ist, kann auch das relativ spröde Verhalten von Knochenzement gegenüber den Wechselbelastungen bei spanender Formgebung umgangen werden.

Im folgenden wird die Erfindung anhand von Ausführungsbeispielen beschrieben. Es Zeigen:
- Fig. 1: Schematisch einen Sagittalschnitt durch eine Tibiaplattform, die über ein erfindungsgemässes Distanzstück aus Knochenzement mit einem Tibiaknochen verbunden ist;
- Fig. 2: schematisch die Zentrierung eines Distanzstückes nach Fig. 1 in Draufsicht;
- Fig. 3a: schematisch die flächige Seite eines Distanzstückes nach Fig. 1 mit vorstehenden Noppen;
- Fig. 3b: schematisch eine vergrösserte Ansicht des Distanzstückes nach Fig. 3a;
- Fig. 4a: schematisch im Sagittalschnitt eine einzementierte künstliche Femurkondyle mit einem erfindungsgemässen Distanzstück für Tiefenkorrektur;
- Fig. 4b: schematisch eine Kondyle nach Fig. 4a mit einem Distanzstück für Korrektur in sagittaler Richtung;
- Fig. 5a: schematisch im Sagittalschnitt eine einzementierte künstliche Femurkondyle mit einem winkelförmigen Distanzstück;
- Fig. 5b: schematisch eine vergrösserte untere Ansicht eines winkelförmigen Distanzstückes gemäss Fig. 5a;
- Fig. 5c: schematisch eine obere Ansicht eines winkelförmigen Distanzstück gemäss Fig. 5b;
- Fig. 6: schematisch einen Schnitt durch eine einzementierte metallische Hüftgelenkschale mit einem schalenförmigen Distanzstück;
- Fig. 7: schematisch für schalenförmige Distanzstücke nach Fig. 6 mit gleicher Innenfläche die exzentrische Variation der Aussenfläche und
- Fig. 8: schematisch für schalenförmige Distanzstücke nach Fig. 6 mit gleicher Innenfläche die konzentrische Variation der Aussenfläche.

Mit den Figuren werden flächigen Distanzstücke für in Knochen zementierbare Prothesenteile gezeigt, die es ermöglichen ein Prothesenteil mit Lagekorrektur seiner Funktionsflächen einzuzementieren, ohne Zusätzliche Risiken beim Zementieren zu erzeugen. Dadurch, dass die Distanzstücke selbst aus ausgehärtetem Knochenzement bestehen, entsteht mit dem flüssigen Knochenzement eine feste homogene Bindung. Gleichzeitig erlauben diese Distanzstücke auch beim Zementieren eine feine, baukastenartige Abstufung und ein exaktes Positionieren von Prothesenteilen.

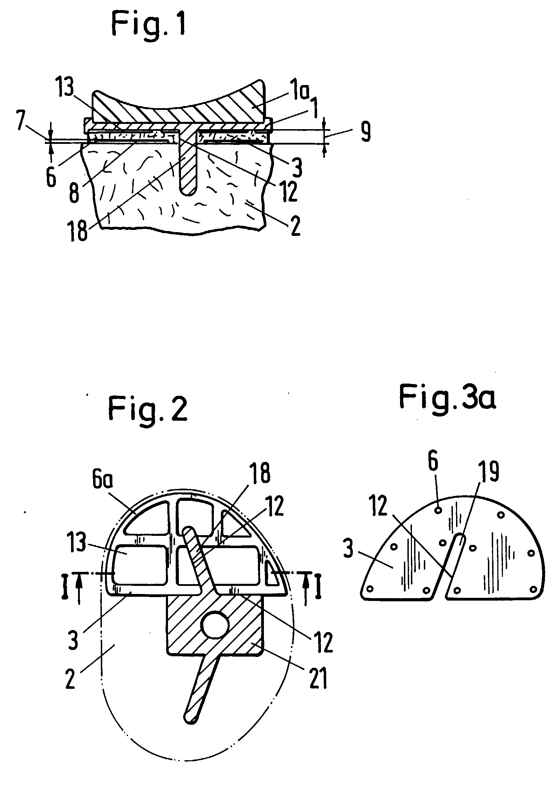

In den Figuren 1, 2, 3a, 3b sind flächige Distanzstücke 3 aus ausgehärtetem Knochenzement gezeigt, die an einer Tibiaplattform 1 und an einem resektierten Tibiaknochen 2 mit flüssigem Knochenzement befestigbar sind. Durch eine baukastenmässige Lagerhaltung von flächigen Distanzstücken 3 mit unterschiedlicher Dicke 9, kann eine auf der Tibiaplattform 1 verankerbare Lagerschale 1a mit ihrer Lagerfläche angehoben werden. Das flächige Distanzstück 3 ist mit Vorsprüngen 6, 6a versehen, die die Dicke 9 des Distanzstückes 3 festlegen und gleichzeitig einen Hohlraum 13 zur Gegenfläche 8 entstehen lassen, dessen Boden mit seinem Abstand 7 die Stärke der Zementschicht zur Gegenfläche 8 festlegt.

In Figur 2 sind mehrere solcher Hohlräume 13 mit einer umlaufenden Kante 6a gezeigt. Ein Teil der Aussenkontur des Distanzstücks 3 und ein Einschnitt 19 bilden Hilfsflächen 12, mit denen das Distanzstück an einer Rippe 18 und an einem Schaft 21 der Plattform 1 zentriert ist.

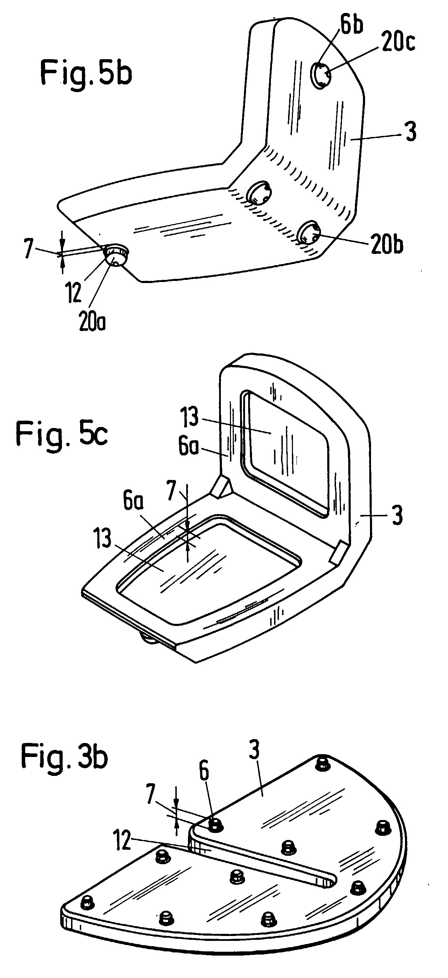

In Figur 3a und 3b ist ein Distanzstück 3 mit vorspringenden Noppen 6 gezeigt, die die Stärke vom Knochenzement festlegen, wenn eine Gegenfläche angepresst wird und der überflüssige Knochenzement seitlich ausgepresst wird.

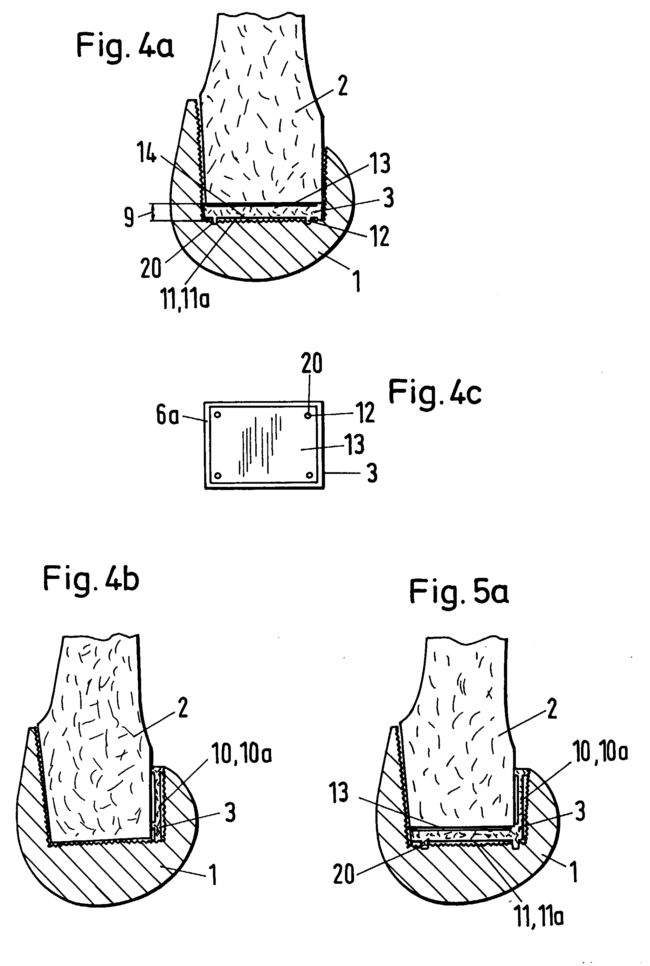

In den Figuren 4a, 4b, 4c sind Distanzstücke 3 für am Femurknochen einzementierbare Kondylen eines künstlichen Kniegelenkes gezeigt, wobei in Figur 4a gegen distal und in Figur 4b gegen posterior unterlegt ist. Die Befestigungsflächen 10a, 11a am Prothesenteil 1 ist so mit einer Struktur 14 versehen, dass eine Verzahnung mit dem Knochenzement stattfinden kann. Die Distanzstücke 3 sind wie in Figur 4c zur Prothese hin mit einer umlaufenden vorstehenden Kante 6a versehen, um die Dicke der Zementschicht festzulegen. In der Nähe der Ecken sind Zentrierstifte 20 angebracht, die über die umlaufende Kante 6a vorstehen und mit vorstehenden zylindrischen Hilfsflächen 12 in Bohrungen der Prothese einfahrbar sind, um das Distanzstück dort zu zentrieren. Die Flächen 10, 11 am Distanzstück und die Gegenflächen 10a, 11a am Prothesenteil sind jeweils eben. Die Dicke 9 des Distanzstückes wird durch die stirnseitig aufliegenden Vorsprünge 6a festgelegt, in Relation zu welchen auch die Tiefe der Hohlräume 13 festgelegt ist.

In der Figur 5a ist eine abgewinkelte Bauform für ein Distanzstück 3 gezeigt, das einer Kombination der Korrekturen in den Figuren 4a und 4b entspricht. Ein für diese Anordnung typisches Distanzstück 3 zeigen die Figuren 5b, 5c. Das abgewinkelte Distanzstück 3 besitzt zum Knochen hin an jedem Schenkel eine umlaufende Kante 6a und einen um einen Abstand 7 tiefer liegenden Boden, mit denen ein Hohlraum 13 zur Aufnahme von Knochenzement gebildet ist. Auf der der Prothese zugewandten Seite sind jeweils verrundete Stifte 20a, b, c angebracht, die an ihrem Fuss eine vorstehende Schulter 6b besitzen, welche mit ihrer Höhe die Zementschicht zur Prothese festlegt. Der unterste Stift wird nach dem Aufbringen des Zements auf der Prothese als erster mit einer zylindrischen Führungsfläche in einer Gegenbohrung der Prothese eingefahren, während die restlichen Stifte 20b und 20c an einer schrägen Uebergangsfläche und an einer um 90° abgewinkelten Fläche nur mit einer schwachen Verrundung über die Schultern 6b vorstehen und so im Rahmen der Elastizität des Distanzstückes 3 in Gegenbohrungen der Prothese einschnappen können. Das Distanzstück ist so selbst bei noch flüssigem Zement zur Prothese hin über die Stifte 20a, b, c mit der Prothese dirigierbar.

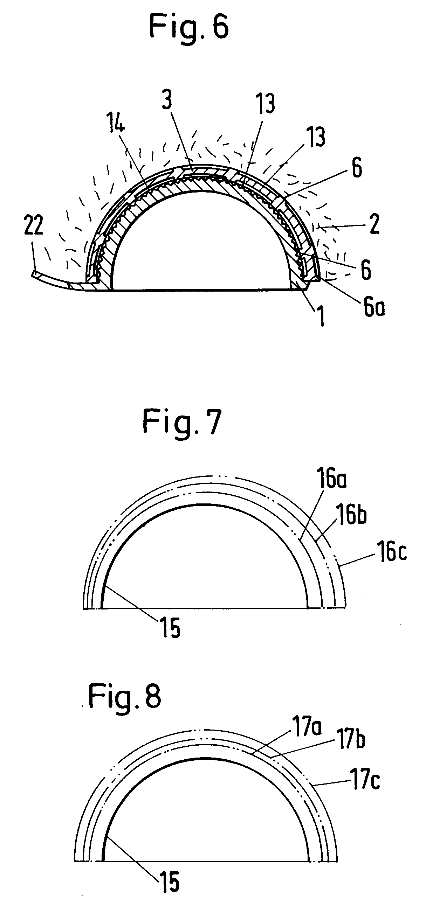

Diese Technik der Distanzveränderung von Funktionsflächen an zementierten Prothesenteilen lässt sich auch auf zementierte Hüftgelenkschalen anwenden. In Figur 6 ist eine metallische Hüftgelenkschale 1 gezeigt, die eine strukturierte Aussenoberfläche 14 zur Verzahnung mit Knochenzement aufweist. Eine aus Knochenzement vorgefertigte äussere Schale 3 besitzt auf ihrer Innenseite und auf ihrer Aussenseite Vorsprünge 6, die auch in Form von umlaufenden Kanten 6a zum Abquetschen von Flüssigzement vorhanden sein können, um begrenzte Hohlräume für flüssigen Knochenzement zu bilden. Der Zement verbindet sich mit dem Distanzstück 3 zu einem homogenen Stück, während er zum Knochen 2 und zur Prothese 1 hin in üblicher Weise zu einer Verzahnung gezwungen wird. Das Distanzstück in Form einer Zusatzschale besitzt eine durch die Vorsprünge 6, definierte Innenfläche und nach Figur 7 entsprechend unterschiedlich weit entfernte nicht konzentrische, ebenfalls durch Vorsprünge 6, 6a definierte Aussenflächen 16a, b, c aufweisen. Mit den nicht konzentrischen Aussenflächen ist zusätzlich eine Verschiebung der Innenschale 3 quer zu ihrer Polachse möglich, während bei einer konzentrischen Anordnung gemäss Figur 8 unterschiedliche Aussenflächen 17a, b, c nur eine Vrschiebung in Richtung der Polachse der Innenschale 3 erlauben. An der Innenschale können zusätzlich Befestigungslappen 22 angeformt sein, die beispielsweise über Schrauben im Knochen verankerbar sind.

## Patentansprüche

1. Baukastensystem für zementierte Prothesen, insbesondere für zementierte Knieprothesen, bei denen ein Prothesengelenkteil (1) mit Knochenzement an einem resektierten Knochen (2) befestigbar ist, dadurch gekennzeichnet, dass es mindestens ein vorgefertigtes flächiges Distanzstück (3) aus ausgehärtetem Knochenzement aufweist, das auf seinen flächigen Seiten (4, 5) mit Knochenzement am Knochen (2) respektive am Prothesenteil (1) befestigbar ist, um den Abstand zwischen Prothesenteil (1) und Knochen (2) zu vergrössern.

2. Baukastensystem nach Anspruch 1, dadurch gekennzeichnet, dass die flächigen Distanzstücke (3) aus PMMA bestehen.

3. Baukastensystem nach Anspruch 1 oder 2, dadurch gekennzeichnet, dass die flächigen Distanzstücke (3) mindestens auf einer flächigen Seite (4, 5) Vorsprünge (6) aufweisen, die eine minimale Distanz (7) der restlichen flächigen Seite zu einer Gegenfläche (8) festlegen.

4. Baukastensystem nach Anspruch 3, dadurch gekennzeichnet, dass beide flächigen Seiten (4, 5) eines Distanzstücks (3) diese Vorsprünge (6) aufweisen.

5. Baukastensystem nach einem der Ansprüche 3 oder 4, dadurch gekennzeichnet, dass die Vorsprünge (6) an den flächigen Distanzstücken (3) als umlaufende Kanten (6a) ausgebildet sind, um flüssigen Knochenzement zur Gegenfläche (8) abzuquetschen und um flüssigen Knochenzement in einem Hohlraum (13) zur Gegenfläche (8) einzuschliessen.

6. Baukastensystem nach einem der Ansprüche 1 bis 5, dadurch gekennzeichnet, dass es für ein Prothesenteil (1) mehrere flächige Distanzstücke (3) mit unterschiedlicher Dicke (9) aufweist.

7. Baukastensystem nach einem der Ansprüche 1 bis 6, dadurch gekennzeichnet, dass die flächigen Seiten (4, 5) von Distanzstücken (3) parallel zueinander stehen.

8. Baukastensystem nach einem der Ansprüche 1 bis 7, dadurch gekennzeichnet, dass die flächigen Seiten (4, 5) von Distanzstücken keilförmig zueinanderstehen.

9. Baukastensystem nach einem der Ansprüche 1 bis 8, dadurch gekennzeichnet, dass flächige Distanzstücke (3) in mehreren Ebenen (10, 11) abgewinkelt sind, um an Gegenflächen (8), die in mehreren Ebenen (10a, 11a) abgewinkelt sind, anliegen zu können.

10. Baukastensystem nach einem der Ansprüche 1 bis 9, dadurch gekennzeichnet, dass die Distanzstücke (3) über Hilfsflächen (12) am Prothesenteil zentrierbar sind.
